# EUROPEAN PATENT SPECIFICATION

(11) **EP 0 742 022 B1**
(45) Date of publication and mention of the grant of the patent: **15.03.2000**
(21) Application number: 94928547.2
(22) Date of filing: 12.08.1994
(51) Int. Cl.: A61L 31/00, A61L 27/00

(54) **BIOLOGICALLY COMPATIBLE HYDROGEL**
BIOLOGISCH KOMPATIBLES HYDROGEL
HYDROGEL BIOCOMPATIBLE

(30) Priority: 10.08.1994 UA 94086726
(43) Date of publication of application: 13.11.1996
(73) Proprietor: MALOE VNEDRENCHESKOE PREDPRIYATIE "INTERFALL", Kiev, 253099 (UA)
(72) Inventor: PAVLYK, Boris Ivanovich, Kiev, 252010 (UA)
(74) Representative: von Füner, Alexander, Prof.h.c. Dr.
(86) International application number: PCT/UA94/00022
(87) International publication number: WO 96/04943

(56) References cited:
- WO-A-89/07455
- GB-A- 1 320 233
- GB-A- 2 114 578
- US-A- 4 540 568
- US-A- 4 631 188
- US-A- 4 873 086
- DATABASE WPI Section Ch, Week 9240 Derwent Publications Ltd., London, GB; Class A96, AN 92-329342 XP002018674 & SU 1 697 756 A (KIEV ORTHOLARYNGOLOGY RES INST) , 15 December 1991
- K.Z. GUMARGALIEVA et al.: "Fizikokhimicheskie Osnovy Sozdaniya Endoprotezov Na Osnove Gidrogelei", (Tezisy), I. INTERNATIONAL CONFERENCE, "Modern Approaches to the Development of the Effective Dressing Materials and Polymer Implantants", 1992, Moscow, pages 211.

## Description

The invention relates to formulations of medical-purpose biocompatible hydrogels intended to be applied as injectable endoprosthesis for mammoplasty (preferably in case of mamma aplasia and hypoplasia), or for phalloplasty, i.e. male sexology (in case of poor erection) for a potency intensification by means of filling corpus cavernosum penis with an elastic medium.

The wish to improve the mentioned and similar cosmetic and functional defects is at present of a mass character and is frequently reasoned by the mere patient's desire.

Numerous hardly-to-be-combined requirements for biocompatible materials of the mentioned purpose should be met, therefore. The major requirements include the following ones:
long-term, preferably life-long retaining of the form and size of an endoprosthetic organ, despite the patient's age, when the former was corrected;
the greatest possible biocompatibility which is, specifically, characterized by the elimination of carcinogenicity, allergic reactions (including the short-term ones immediately followed by the injection of selected material into an organism or its application at the skin and mucous membrane in particular), absence of an expressed encapsulation or rejection of endoprosthesis and free-occurred metabolic processes in the region filled with a biocompatible material;
minimal traumaticity and prolongation of a biocompatible material injection, especially under endoprostethics with a high-dose (up to 1 l) application.

Individual meeting of the requirements mentioned or their combinations are of no special problem.

Thus, using glycerol-based fluoroplastic (teflon) paste as a biocompatible material for larynx endoprosthetics, considerably stable clinical effect may be obtained (see: Beck Ch.L. Unsere Erfahrungen mit der intralaryngealen Tefloninjection // Laryngol. Rhinol. Otol. - 1980. Bd. 59 - No. 11. - S. 715-718; Lewy R.B. Teflon injection of the vocal cord: Complications, errors and precautions // Ann. Otol. - 1983. Vol. 52. No. 5. pp. 1, 473-474; Berghans A. Verfahren zur Unterfütterung von Stimmlippen // H.N.O. - 1987. Bd. 35. No. 6. S. 227-233).

In comparison with water, glycerol is known to have higher viscosity; therefore a paste stored is considerably stable. When injected, glycerol serves as an effective lubricant.

Due to its unlimited solubility in water and water-containing liquids of an organism, however, glycerol is quite rapidly (few hours to one day) removed from the endoprosthetic region. Subsequent gradual mechanical removal of teflon microparticles, leaving zone together with lymph - and bloodflow, is possible. It results in the prosthesis volume lowering and considerable decrease in the treatment efficacy. Therefore, despite the teflon biochemical inertness, it should be repeatedly applied.

Besides, the teflon hard particles damage tissues, contacting with the endoprosthetics, in a mechanical way. Actually in all cases, initially it causes an expressed aseptical inflammatory reaction and, sometimes, laryngostenosis with a possible urgent tracheotomy required.

Application of gel-forming biocompatible materials for the mentioned requirements is, therefore, more advisable.

In fact, minimal traumaticity, endoprosthetics prolongation, absence of carcinogenicity and minimal allergic reactions are obtained by using water-solution of bovine collagen, which is highly refined and degraded by the degree of polymerization. Being injected into form-and-size corrected organ, collagen at the temperature below 37° C forms an elastic and mechanical-deformation-resistant hydrogel (see: Ford Ch., Martin O.M., Warner Th.F. Injectable collagen in laryngeal rehabilitation // LARYNGOSCOPE, 1984. 94. pp. 513-518).

As a protein, however, collagen in considerably short period (less than half a year) is completely resorbed in the patient's organism. It is, therefore, suitable for endoprosthetics primarily in the cases when a complete connective tissue substitution for endoprosthesis is acceptable or when a patient, according to medical indications, is required exactly a temporary endoprosthesis.

Considering the above stated, it should be more reasonable to give preference to gel-forming biocompatible materials based on synthetic polymers.

Thus, a biocompatible gel-forming material in the form of hydrophilic polyglycols of metacrylic acid esters is known to be applied for endoprosthetics (Kresa L., Rems T., Wichterle O., Hydrogel implantat in vocal cord // Otolaryngol. Head Neck Surg. - 1988. V. 98. No. 3 pp. 242-245).

Required dose of the given dry-form material is implanted via section in the region of cosmetic or functional defect correction and an operative wound is sutured. Absorbing water from the adjacent tissues, the material swells thereupon and promotes a local increase in corrected organ volume, e.g., a vocal cord.

The mentioned biocompatible material possesses a high biochemical stability.

When applying it, however, the durable therapeutic effect is obtained at the expense of traumatic surgical interventions associated with edemas and aseptic inflammations.

Biocompatible gel-forming materials, ready-made liquids that could be used as injections, are, therefore, the most promising for endoprosthetics.

A biocompatible gel-forming material in the form of a water-insoluble solution of non-cross-linked polymer or acrylonitrile copolymer, polyvinylacetate, linear or low-branched polymers and 2-hydroxyethyl-acrylate copolymer and methylacrylate, n-vinyliminocarbonyl polymer in dimethyl sulfoxide or other polar water-free-miscible organic solvent, may be cited as example (Stoy V., Chvapil M. US Patent No. 4631188, 1986). Obtaining copolymers, as the additional monomers, are intended to use, namely: acrylamide (including N-substituted), acrylhydrazide (including N-substituted), acrylic acid and acrylates, glutarimide and vinyl sulfone and, as the polar water-free-miscible solvents, glycerin and its mono- or diacetates, methanol, ethanol, propanol and isopropanol, dimethylformamide, glycols, etc.

The said material is highly efficient for correcting slight cosmetic or functional defects, specifically, for endoprosthetics of lips and other parts of the face, and vocal cords.

For correcting the mamma forms and sizes by endoprostheses, up to 1 l of the mentioned material may be required, however. In similar cases, quantity of an organic solvent, injected with gel-forming polymer, substantially exceeds the physiologically permissible minimum, what may result in the occurrence of an erythema and, in certain cases, an allergic shock. Simultaneously, due to the linear structure of the gel-forming polymer applied, a low form-stability of the endoprostheses is observed: the higher volume resulted in lower quality.

Thus, applying allergen-free ready-made hydrogels is most preferable.

GB 2 114 578 discloses a polyacrylamide gel for medical and biological purposes containing a copolymer of acrylamide and methylene-bis-acrylamide, wherein the polyacrylamide gel further contains a physiological solution, the content of the component being (% by mass):

| | |
|---|---|
| polyacrylamide | 3.0 to 28.0 |
| physiological solution | 72.0 to 97.0. |

The polyacrylamide gel can be used as a base of nutrient mediums for growing microorganisms, artificial crystalline lenses and elastic contact lenses.

A biocompatible gel, containing 3.0% by mass of polymer, based on acrylamide, which is obtained by using a free-radical polymerization initiator (specifically, ammonium persulfate) in a dispersion medium in the form of pyrogen-free bidistilled water (see the Specification of SU 1697756).

The said hydrogel is of actually complete biocompatibility with the human organism's tissue and media by all of the above-mentioned aspects and may, therefore, be applied at considerable (up to 1 l) volumes, causing no expressed negative biochemical and biological aftereffects. In the region of injection (endoprosthesis) it forms a structure, free-to-be-permeable not only by water, ions and oxygen, but by low-molecular metabolites as well. Implants of the hydrogel described intergrow with the recipient's own young fibrous tissue at a considerably rapid rate (by the fifth-sixth month). The given result is especially valuable in vocal apparatus alloplasty.

The hydrogel described is, however, of low viscosity and, subsequently, low elasticity and high mobility. As the bond between water and the polyacrylamide macromolecules is loose, the former is rapidly removed from implants which results in their distinct shrinkage and considerable decrease in cosmetic or therapeutic effect. Thus, in case of volume (e.g., intra-mammar) endoprosthetics, the implants' resistance to external deformation loads and shrinkage is inversely proportional to their initial volume.

The invention is, therefore, based on the problem to provide a biocompatible gel with higher elasticity, form-firmness and stability of massive implants and, thus, more valuable therapeutic or cosmetic effect for the use in endoprosthetics.

The subject matter of the invention is the use of a biocompatible hydrogel containing 3.5 to 6.0 % by mass of a polyacrylamide, which is cross-linked with methylene-bis-acrylamide, and pyrogen-free water as dispersion medium for the preparation of an injectable endoprosthesis for mammoplasty or phalloplasty.

The hydrogel, retaining its ability to be permeable for water, ions, oxygen and low-molecular metabolites and suitability to be applicable as injection, has a more regular and more advantageous water-binding structure which provides obtaining massive, highly elastic and form-stable implants (e.g., intramammar endoprostheses, supporting bars in corpus cavernosum penis), which are sluggishly (some months to some years) being grown out by a soft rich-vascularized fibrous tissue. Due to the mentioned structural, biochemical and anatomic-physiologic advantages, the cosmetic and/or therapeutic effect of endoprosthetics as well as the stability of the given effect in time, become more favourable.

Preferably, the biocompatible hydrogel includes a cross-linked polyacrylamide obtained by using methylene-bis-acrylamide as cross-linking agent and a mixture of ammonium persulfate and tetramethylethylenediamine as polymerization initiator. Methylene-bis-acrylamide is an analogue to the base monomer (acrylamide) both by its composition and biocompatibility. Application of the polymerization initiator mixture favors the marked regular cross-linking of polyacrylamide chain macromolecules in an elastic space network, suitable for the hydrogel injection.

The biocompatible hydrogel contains 3.5 to 6.0% by mass of the cross-linked polyacrylamide. This range of concentration is the maximum favorable for therapeutic or cosmetic effect in injection endoprosthetics. At concentrations lower than 3.5%, the hydrogel is unstable and may be applied as medicinal ointments base or electroconducting immersion media for cardio- or encephalography. At a concentration higher than 9.0%, it virtually loses its viscosity and, in certain cases, may be used for manufacturing the relatively firm form-rigid, preliminary cast endoprostheses, implantation of which requires an operation access to the prosthetics region.

Efficiency of the biocompatible gel suggested as applied for mammoplastics is illustrated by two computer tomograms, viz.:
Fig. 1 - illustrates the result of correcting form and size of the right mamma and
Fig. 2 - the results of the similar correction of the left mamma in patient K.

The essence of the invention is thereafter revealed as follows:
by description of the initial reagents, method of obtaining, examples of the method realisation and results of laboratory tests realisation and results of laboratory tests of the biocompatible hydrogel suggested,examples of the biocompatible hydrogel composition, description of methods and results of chemical, biochemical and medical studies of the biocompatible hydrogel suggested.
description of the methods for correcting cosmetic and functional defects of human organism by the intended injections of the biocompatible hydrogel suggested, and information on its practical application.

To obtain the biocompatible gel suggested, the following reagents were used, viz. (see Table 1. p. 9).

Except the bidistilled water, reagents of "REANAL" firm (Hungary) were used in the experiments, namely: acrylamide and methylene-bis-acrylamide in the form of white crystals, tetramethylethylenediamime in the form of white viscous liquid and ammonium persulfate in the form of colorless crystals.

Conventionally, the biocompatible gel suggested is obtained by the following method:
under aseptic laboratory conditions, calculated amounts of acrylamide and water-diluted solutions of the cross-linking agent, i.e. methylene-bis-acrylamide and initiators of polymerization, e.g. ammonuim persulfate and TMED, are placed into a sterile glass container. The given reagents are intimately mixed, then diluted with water (physiological solution or other water-diluted physiologically neutral salt, e.g. sodium acetate); the mixture is then filtrated and the filtrate is allowed to stand for obtaining the cross-linked polyacrylamide (CL PAA) hydrogel.

The ready-made CL PAA hydrogel is controlled for the following characteristics:
- appearance, as according to visual evaluation;
- the hydrogel should have no color or impurities containing;
- refraction index should be within the range of 1.334 to 1.350;
- pH should be within the range of 7.0 - 9.0;
- heavy metal contents should be less than 0.001% by mass;

**Table 1**

| DATA ON REAGENTS FOR OBTAINING THE BIOCOMPATIBLE HYDROGEL SUGGESTED | | |
|---|---|---|
| Name and empirical formula of reagent | Ammount required for 100g hydrogel, g | Indices controlled, units for their measuring and values tolerable |
| Acrylamide | 3-5 - 6.0 | Melting temperature, °C, |
| C₆H₅NO | | 84.5+/-0.5 |
| | | Density, g/cm³, 1.222 |
| | | Content of the ground substance, % mass, at the least 98 |
| Methylene-bis-acrylamide | 0.01 - 1.00 | Melting temperature, °C, 184+/-1.0 |
| C₇H₁₀N₂O₂ | | Content of the ground substance, % mass, at the least 96 |
| TMED - tetramethylethylendiamine C₆ H₁₀N₂ | 0.001 - 1.00 | Density, g/cm³, 0.78 Content of the ground substance, % mass, at the least 98 |
| Ammonium persulfate | 0.001 - 1.00 | Density, g/cm³, 1.98 Breaking temperature, °C, 120 |
| (NH₄)₂S₂O₈ | | Content of the ground substance, % mass, at the least 98 |
| Bidistilled pyrogen-free | the rest to 100 | Refraction index, 1.3329 |
| water | | |
| - sterility. | | |

Examples sited below will provide more detailed illustration of the substance of invention.

### Example 1. Preparation of the low-concentration biocompatible hydrogel

In 1-l glass container 20.3 g acrylamide, 8.7 ml 2% methylene-bis-acrylamide aqueous solution, 7.5 ml 4% TMED aqueous solution and 15 ml 4% ammonium persulfate aqueous solution were mixed. The mixture, using water, has been brought to the end volume of 580 ml; then it was filtrated by the glass filter and the filtrate was allowed to stand for at least 20 minutes until 3.5% CL PAA hydrogel was formed.

### Example 2. Preparation of the medium-concentration biocompatible hydrogel

In 1-l glass container 24 g acrylamide, 50 ml 1%- methylene-bis-acrylamide aqueous solution, 25 ml 1%- tetramethylethylenediamine aqueous solution and 50 ml 1.3%- ammonium persulfate aqueous solution were mixed. The mixture has been brought to the end volume of 350 ml by using water; then it was filtrated by a glass filter and the filtrate was allowed to stand for at least 20 minutes until 5%- CL PAA hydrogel was formed.

### Comparative Example: Preparation of the high-concentration biocompatible hydrogel

In 1-l glass container 34.2 g acrylamide, 60 ml 1%- methylene-bis-acrylamide aqueous solution, 6 ml 1% TMED aqueous solution and 25 ml 0.48%- ammonium persulfate aqueous solution were mixed. The mixture has been brought to the end volume of 380 ml by using water; then it was filtrated by a glass filter and the filtrate was allowed to stand for at least 20 minutes until 9% CL PAA hydrogel was formed.

In experiments the following compositions of the CL PAA biocompatible hydrogel (BCH) were used (see Table 2, page 11).

As shown in the Table, Examples 2 and 3 are in conformity with the limiting values of the CL PAA concentration in the hydrogel.

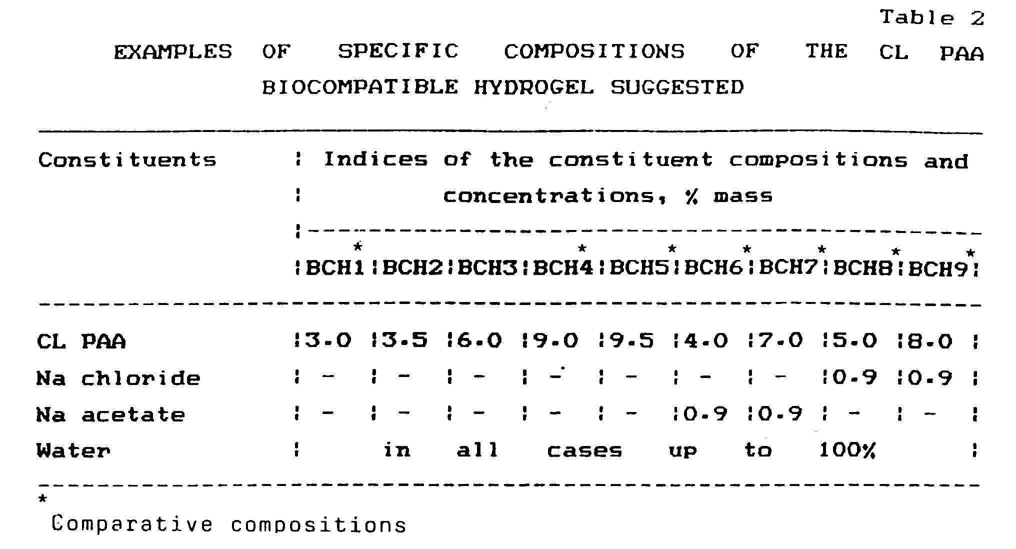

Laboratory studies of the hydrogel suggested were performed chemically, biochemically and medico-biologically. The given distinction was not so strict; it was virtually based on methods and techniques applied.

Thus, studies of the dry residue, which is conventionally used to determine a precise concentration of a definite substance in true or colloid solution, were conducted.

The given method was applied for evaluating chemical stability of the hydrogel suggested.

Hydrogel, containing relatively slightly-cross-linked (0.25% methylene-bis-acrylamide of the acrylamide mass) CL PAA with a calculated concentration of about 5%, was prepared. Four series of five samples of the given hydrogel, each volume about 20 ml, were treated as follows:
the 1st series - weighed and dried at a temperature of 35°C and residual pressure of 12 - 15 mm Hg to constant mass (for about 20 hours);
the 2nd series - weighed and poured with bidistilled water, boiled for 15 minutes and dried afterwards, as it was mentioned above, to constant mass;
the 3rd series - weighed, each poured with bidistilled water up to 200 ml level, steeped for 7 day (water being changed every day) and dried, as it was stated above, to constant mass;
the 4th series - weighed, steeped for 7 days, similar to samples in the 3rd series, boiled for 15 minutes, similar to samples in the 2nd series, dried afterwards, as it was stated above, to constant mass.

By means of conventional computing for each sample the polymer percentage in the hydrogel mass was determined.

The results obtained were as follows (see Table 3):

**Table 3**

| RESULTS OF STUDIES OF CHEMICAL STABILITY OF THE SUGGESTED CL PAA BIOCOMPATIBLE HYDROGEL WITH THE DRY RESIDUE DETERMINED | | |
|---|---|---|
| Series | Sample average mass, g, and peak deviation from the average | |
| | Before treatment | After treatment |
| 1 | 20.84+/-0.96 | 0.983+/-0.0048 |
| 2 | 20.15+/-0.87 | 0.951+/-0.0076 |
| 3 | 20.65+/-0.83 | 0.923+/-0.0065 |
| 4 | 20.41+/-0.63 | 0.913+/-0.0095 |

As follows from the results obtained, steeping with subsequent boiling actually causes no destruction of the CL PAA in the hydrogel and testify to its potential, as it would be required, thermal sterilization, as well as to stability of even the slightly cross-linked CL PAA.

Consequently, using the suggested CL PAA hydrogel stability in aqueous medium, the ability of the acrylamide to migrate into biotissues was evaluated.

The given index was determined by the method of highly efficient liquid chromatography (HELC) with detecting the absorption of ultraviolet radiation in the range of 195 nm, which is typical of the monomer mentioned; chromatograph "Liquochrom" (Hungary) has been applied thereby.

Thus, by steeping the samples of the hydrogel suggested throghout 14 and 30 days at a temperature of 40°C and ratio of 100 ml extractant (bidistilled water) - 1 mg gel, the extracts were obtained. Samples for the HELC have been prepared as follows: the extract aliquot doses of 5 ml were dried under a residual pressure of 12 - 15 mm Hg and at room temperature; residue then was single-eluated at a rate of 0.2 ml/min with a 2 ml water/methanol mixture (ratio 1:1); a column of 150 mm - length and 4 mm - diameter with the SEPARON C18 phase was applied and eluate being feeding into a loop of the 20 µl - volume injector.

By the HELC-method, the minimal detectable concentration of acrylamide is 0.000001 mg/l and its limiting permissible concentration (LPC) in aqueous solutions from materials, applied for implants, is 0.02 mg/l.

In aqueous extracts from hydrogels, obtained by the methods described, acrylamide is not detectable, which serves as an evidence for high chemical stability of the CL PAA as well as biocompatible hydrogel suggested as a whole.

In medical-biological aspects, samples of the CL PAA hydrogels, obtained by the method described, have been tested under laboratory condition for:
biochemical and hemolytic activity;
embriotoxic activity;
mutagenous activity;
carcinogenic activity.

Biochemical and hemolytic activity of the CL PAA hydrogels was evaluated by alteration in indices in albino male-rats of the "Vistar" line, mass of 300-350 g, experimental and control groups each consisting of 16 animal units.

At the start of the experiment,the control narcotized rats were intraperitoneally syringed with the 5%-hydrogel suggested.

Rats of both groups were kept on a conventional diet.

Two weeks afterwards using the biochemical analyzer (production of "CORNING" Firm, Sweden), rats were taken blood to determine its components as follows: Na, K, Ca and Cl ions; urea, blood urea nitrogen, uric acid nitrogen; creatinine and enzymes (amylase, alkaline phosphatase, alanine - and aspartate aminotransferase, thereon being stated as AlAT and AsAT, respectively; lactate dehydrogenase (LDG) and creatinine were determined by the bio-assay "Lachema" (Chekhia). Results obtained are shown in Table 4 (p. 14).

**Table 4**

| EFFECT OF IMPLANTS MADE OF THE CL PAA BIOCOMPATIBLE HYDROGEL SUGGESTED ON BIOCHEMICAL BLOOD COMPOSITION IN RATS | | |
|---|---|---|
| Biochemical indices and units for their measuring | Results of measurement | |
| | in the controls | in the experiments |
| Sodium, mmol/l | 151 | 148 |
| Potassium, mmol/l | 8.20 | 6.82 |
| Calcium, mmol/l | 0.97 | 0.90 |
| Chlorides, mmol/l | 97.5 | 102.1 |
| Urea, mmol/l | 4.8 | 4.8 |
| Blood urea nitrogen, mmol/l | 2.2 | 2.2 |
| Creatinine, mmol/l | 0.05 | 0.05 |
| Amylase, mg % | 89.1 | 83.33 |
| Alkaline phosphatase, mmol/l | 84.5 | 55.9 |
| AsAT, mmol/l | 133 | 130 |
| AlAT, mmol/l | 41 | 51.7 |
| LGD (total), mmol/l | 217 | 189 |
| Creatinine phosphokinase, un. | 5960 | 5685 |
| Uric acid, mmol/l | 0.14 | 0.1 |

As it follows from the Table, the major electrolytes indices show the absence of marked injuries of the cell-membranes. The ATP-ase activity is also normal-

Stability of nitrogen metabolism testifies to the normal metabolism, including purine metabolism; the latter, being combined with the creatinine stability, proves a stability of functioning the excretion system, when the CL PAA is present in organism.

AlAT and AsAT normal activity indicates to the hepatocytes stability and the adequate state of cardiac muscle, which, considering a normal creatinine-phosphokinase activity, is not subjected to considerable overloadings.

Sufficient alkaline - phosphatase activity shows the absense of inflammation processes in the biliary duct endothelium.

In addition, blood cell analysis of the rats mentioned, was conducted and the results are shown in Table 5.

**Table 5**

| EFFECT OF IMPLANTS MADE OF THE CL PAA BIOCOMPATIBLE HYDROGEL SUGGESTED ON BLOOD COMPOSITION IN RATS | | |
|---|---|---|
| Indices of blood cells composition and units for their measuring | Results of measurement | |
| | in the controls | in the experiments |
| Leukocytes, thsd/µl | 3.5+0.2 | 5.4 |
| Erythrocytes, mln/µl | 6.86+0.43 | 7.02+0.31 |
| Hemoglobin, g/l | 125+12 | 136+9 |
| Hematocrit, % | 35.0+1.5 | 36.5+1.3 |
| Erythrocytes mean diameter, nm | 51.0+0.2 | 52.0+1.5 |
| Hemoglobin average content in one erythrocyte, pg | 35.7+0.3 | 38.1+0.5 |
| Thrombocytes, thsd/µl | 992+12 | 694+50 |
| Thrombocytes mean diameter, nm | 8+1.5 | 14.25+1.6 |

As it follows from Table 4, leukocyte amount in the experiment is inconsiderably over the norm of 4.5 thsd/µl and data, showing blood erythrocyte concentration and erythrocytes hemoglobin, are an evidence for adequate blood oxygenation- In case of hematocrit, one may testify that water-salt equilibrium is about the norm.

Indirectly, all information available confirm a high biochemical stability of the CL PAA itself as well as its high biocompatibility.

Embryotoxic activity of the CL PAA hydrogels was determined in an experiment; three groups of albino breed-free female-rats, mass of 180 - 200 g, each consisting of 16 animal units, were used.

Rats of the first group were intraperitoneally syringed with 2 ml 5%-hydrogel suggested in a week they were to be served

At the third day of being pregnant, rats of the second group were, in the similar manner, intraperitoneally injected with 2 ml 5%-hydrogel suggested.

Pregnant intact rats consituted the third group.

Two rats in the first group showed no pregnancy. The 14 rats in the first group and all of the 16 rats in the 2nd and 3rd groups gave birth to normal healthy cubs that proves absense of embryotoxygenicity of the hydrogel suggested.

CL PAA hydrogels mutagenicity was studied, using bone marrow reticulocytes of the C3H1-line mice (of both sexes) two-months of age in two groups, each consisting of 10 animal units.

Mice in the control group were injected with the 30-days aqueous extract (0.01% by body mass) from the 9%-CL PAA (comparative) hydrogel, the former being prepared at a temperature of 40°C and a ratio of 100 ml extractant/1 g gel.

In 24 hours the experimental and intact mice were killed by means of spinal marrow shift; bone marrow smears were prepared by known methods, using a serum of fresh non-stored human blood group AB(IV); Pappenheim's method of staining was applied thereby.

By microscopic study, the number of reticulocytes, containing micronuclei, was counted. It has been determined that the difference in numbers of the given reticulocytes between bone marrow smears in the experimental and intact mice, when being counted in 20 visual fields, each, by 1000 cells, did not exceed 2.3%, which is an evidence of the CL PAA hydrogel non-mutagenic effect.

Carcinogenecity of the CL PAA hydrogel was evaluated by the method of immunodetection of organ-specific tumor-associated antigens.

The mentioned method (in said version) is based on determining the electrophoretic mobility (EPM) of stabilized and tanninized erythrocytes, which are sensibilized to tumor-associated antigens of rhabdomyoblastoma and, additionally, to a non-specific embryonal antigen; the latter, in a positive reaction, serves as an indicator of tumor progressive growth. Generally, the EPM-tests are considered to be positive, if the electrophoretic mobility of cells-indicators is decreased by 20% or more.

Experiments conducted have included 12 albino line-free male-rats, mass of 180 - 200 g, divided in to experimental and control groups, each consisting of 6 animal units.

Rats in the control group, being locally anesthetized, were musculus femoris - injected with 4 ml 6% CL PAA hydrogel. Then, for 18 months the rats were kept on their usual diet. Afterwards, all animals were taken blood from tail vein; erythrocytes were isolated from samples and sensibilized on the mentioned antigens; the EPM-test were carried out.

Slowing down of the EPM-sensibilized erythrocytes as compared with the non-sensibilized ones, expressed as follows:
4.17+/-1.58% in rats of the experimental group for rhabdomyoblastoma antigen and 1.67+/-0.95% for non-specific embryonal antigen, and
1.5+/-0.62% in rats of the control group for rhabdomyoblastoma antigen and 1.83+/-1.28% for non-specific embryonal antigen.

Thus, the EPM-test appeared to be negative for rats of both groups, which testifies to the absence of carcinogenous activity of the CL PAA hydrogel suggested.

The most detailed medico-biological studies of the CL PAA biocompatible hydrogel suggested as applied for endoprosthetics and tamponing in medical practice, were conducted on breedless three- /four-year-old male-dogs, mass of 25 to 30 kg; under sterile conditions, in locally anesthetized dogs, after the skin of penis was disinfected with 10%-iodine tincture, an endoprosthetics has been modelled, viz:
in 6 dogs, subcutaneously, 5 ml 3.5%-CL PAA hydrogel was single-injected;
in the next 6 dogs, endofascially, excluding penetration under tunica albuginea corporum cavernosum, in the three segments on each side along the penis, the 9%-CL (comparative) PAA hydrogel was injected in an amount up to 1.5 ml per segment, total dose being 8.0 ml, and
in another 6 dogs, intracavernously, including penetration under tunica albuginea, mainly in trabecula corporum cavernosum but excluding injury of an urethra, the 6%-CL PAA hydrogel was also injected in the three segments on each side along penis in an amount up to 1-5 ml per segment, total dose being 8.0 ml.

The fourth group was the control group consisting of three dogs.

Each dog was killed by intravenous nembutal injection:
in the experimental groups in 1, 7 and 14 days and 1, 3 and 6 months after the CL PAA hydrogel suggested was implanted;
in the control group in 1, 3 and 6 months.

Excited pieces of the complete cross section of penis, regional lymph nodes and canine lungs, together with the control section, were fixed in 10% and 6% neutral formaline and the Carnua liquid, dehydrated in alcohols of increasing strength and poured with paraffin.

Mounts were stained with hematoxylin and eosin, pyrofuchsin by van Gison; for detection of glycosaminoglycanes, at the different pHs of staining solution, applying chemical and enzymatic control, the mounts were stained on elastic by Weigert using toluidine blue.

Glycoproteides and glycogen were detected by means of the PAS-reaction by Mac-Manus; calcium salts were detected by von Kos; RNA was detected by Brashe (the control with ribonuclease).

Activity of the following enzymes was studied, namely:
malate dehydrogenase (MDG);
succinate dehydrogenase (SDG) - by Nachlas;
lactate dehydrogenase (LDG);
glucoso-6-phosphate dehydrogenase (G-6-PDG). NAD - and NADFdiaphorase, respectively, by Hess, Scarpelli and Pearce;
alkaline phosphatase (AP) by Gomori, and
adenosintriphosphatase (ATPase) by Wachstein-Maisel.

Nervous tissues were silver-nitrate impregnated by the Bilshovsky-Gross method.

Histo-chemical reactions and controls for them were conducted as recommended in the Manual by E.Pearce (Histochemistry / transl. from English / 2nd Ed.-M: 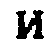 Λ. 1962).

Studies showed the following:
A.When the CL PAA hydrogel was subcutaneosly injected:
   in 1 day in the site of injection a socked-like swelling of soft-elastic consistency with a slight skin thinning was observed (one dog developed inconsiderable edema and hyperemia of tissues, adjacent to an implant, a small focal hemorrhage being resolved by the 7th day, which can be considered as a manipulation injury);
   in 7 days the hemodynamic, alternative or inflammation reactions were not observed- Being studied histologically, the implant had an appearance of a large light-blue vacuole, surrounded by a thin connective-tissue capsule, which separated the CL PAA hydrogel from penis and dermis fascial. Capsule consisted of one-two layers of young fibroblasts encircled by the gentle collagen and elasticfibers. Pyroninophilia and enhanced activity of the reduction-oxidation enzymes (SDG, MDG, NAD- and NADF-diaphorases, LDG) and AP are typical for fibroblast cytoplasma- The G-6-PDG increased activity testified to activation of metabolism pentose-type- Low-dense leukocyte and macrophage infiltration was observed in the near-to-surface layer. Granulation tissue along the capsule periphery was constituted of the new-formed vessels, covered with swelled epithelial cells, lumens of the former being enlarged and filled with the blood formed elements. Proliferating fibroblasts, histiocytes and solitary plasmatic cells were detected in the vessels adventitia. In all cases a giant-cell reaction did not occur. At all pHs of the solution applied metachromatic foci, when stained with the toluidine blue, were not detected. Definite nervous fibers, being impregnated with silver nitrate, showed various alterations, i.e.: local swelling of axis cylinders, their fibers disintegration, vacuolization, varicosity, hypo - or hyperimpregnation. Oxoplasma excrescence, along the nervous fibers or at their ends, partial disoders of the smooth myeline membrane and its decomposition into short and long fragments were observed in some cases. Changes mentioned, are typical of the compensatory-adaptive re-structure of nervous fibers as a response to a compression caused by vacuole on the CL PAA hydrogel;
   in 14 days the macrophage-leukocyte reaction around the CL PAA hydrogel implant was slightly increased; marced fibroblastic reaction, including formation of connective-tissue capsule around a vacuole, occured; the former, at certain sites, was represented by the randomlocated collagen and elastic fibers, having young fibroblasts and newly-formed capillaries in the interspace, and at other sites it was represented by the more mature connective tissue, consisting of some layers of parallel-collage- and elastic fibers as well as proliferating fibroblasts. In the cytoplasm and nucleolis the RNA-content, activity of reduction-oxidation and hydrolytic enzymes was increased. Fibroblasts cytoplasm was rich in metachromatic granules, well-detectable with toluidine blue at pH=2.8, which is typical, when the glycosaminoglycanes synthesis is increased. Capsule-surrounding tissues had a considerably decreased number of the newly-formed vessels, and the histiogenous-type cells, producing glycosaminoglycanes and collagen, were prevailing at their sites. Giant cells were extremely infrequent to occur. Alterations in the nervous fibres were similar to those described above;
   In 1 month after injection a mature connective tissue capsule was formed around vacoule of CL PAA hydrogel which constisted of circle-located collagen and elastic fibres with mature fibroblasts within intervals containing a moderate number of RNA-content and highly-sulfated detectable toluidine blue at the pH = 2.8 glycosamineoglycanes. Activity of reduction-oxidated and hydrolytic enzymes in cytoplasm of fibrocytes is normal. Sometimes a cellular reaction had been seen on the surface of hydrogel like an inconsiderable diffusive infiltration caused by macrophages and plasmatic cells. Fibre structure, suurounding implants, was completely normalized and had not differed from the structure of similar tissue of intact animal. Rective alterations of sensitive nervous tissue are decreasing and are expressed mainly by the non-uniform regions of expansion or thinned axial cylinders and their hypo- and hyperimpregnation;
   in 3 months there was noted a slight thickening of the CL PAA hydrogel with increased basophilic character as well as good separation of implant from adjacent tissues of connective-tissue capsule from collagen and elastic fibres and cells of fibrocytes-type. Structural and histochemical alterations in adjacent tissues were absent and nervous tissues gained normal forms;
   in 6 months the form and size of the implant were practically similar to those as on the first day after injection of CL PAA hydrogel. Histologically, the implant had an appearance of solid well-capsulated dark-blue vacuoles. Capsule consisted of one-two layers of fibrocytes and well-located collagenic and elastic fibers in which calcium salts had not been detected by von Kos method. Neither reactive, nor hemodyanamic, nor dystrophic, nor necrotic, nor inflamatory, nor other changes had been detected in Implant-surrounding tissues, including tissue and cellular atypism. Nervous tissues while impregnated with silver nitrate were in norm.
B. When CL PAA hydrogel was injected endofascially:
   in 1 day and in 7 days penis had uniformly-thickened appearance and heightened elasticity.
      Dogs body temperature was normal, skin coloring at the injection sites was conventional, local inflammations were absent. Histologically, the implants were detected in the sites of injection in the form of light-blue vacuoles. In seven days the CL PAA hydrogel vacuoles were surrounded by thin-walled capsules, mainly consisting of one-two layers of young fibroblasts and encircled by gentle connective-tissue-newly-formed capillaries; leukocytes and macrophages were observed at the hydrogel surface. In the cytoplasm and fibroblast nucleoli the RNA content was increased as well as activities of the SDG, MSG, NAD- and NADF-diaphorases, LDG and G-6-PDG in the cytoplasm were more effective, too. Newly-formed capillaries with slightly widened and blood-filled interspaces and swelled endothelium composited the granulation tissue surrounding the capsule. Proliferation fibroblasts, impured with plasmatic cells, were foud in blood vessels adventatia. No dystrophic or necrobiotic changes were detected in the fascia parted tissue, adjacent to implant. Thus, metachromasy foci, which would testify to the CL PAA hydrogel destruction were not found at staining mounts by toluidine blue at any pHs of its solutions. Permeability of vessels remained normal, as the PAS-positive material, stable to amylase and in perivascular spaces as well as in walls of small and middle vessels, and the AP and ATP-ase in the walls of microcirculatory bed remained low. In some cases, the nervous fibres, impregnated with silver nitrate and studied by Spielmeier, were wave- or spiral- shaped, and, in definite cases, had swells at their ends. Regions of demyelination were rare to observe as well as a local spreading of nervous fibers with loop-like structures formation. Infrequent proliferation of the hypertrophic Schwann's cells was observed. Changes mentioned should be considered as nervous fibers' response to compression by implants;
   in 14 days the macrophage reaction nearby the implants was slightly more intensive but giant cells were not observed however- Distinctive fibroblast reaction, including formation of the connective tissue capsules around vacuoles, was observed; capsules, in the definite regions, were represented by randomly scattered collagen and elastic fibers with young fibroblasts in interspaces, having high RNA content in the cytoplasm and enhanced reduction-oxidation activity of enzymes. More mature connective tissue of the sequenced collagen and elastic fibers and fibroblast-type cells were determined in other regions. Increase in number of histogenous cells and decrease in newly-formed vessels were observed in the granulation tissue, adjacent to a capsula. Endothelium and middle membrane structures, vessels adventitia and hemodynamic indices were unchanged but the nervous cells changes were similar to those as described for the previous term;
   in 1 month the implant capsules consisted of cell elements of fibroblastic sequence, fibrocytes with a mederately pyroninophilous cytoplasm being prevailed- When staining with methylene blue at pH=2.8, a moderate number of high-sulfated glycosaminoglycans in fibroblasts were determined- Enzyme activity in the fibrocytes cytoplasm was in accordance with the control. Undersurface layer of the CL PAA hydrogel was inconsiderably infiltrated by macrophages and plasmatic cells. Neither blood-flow disoders, inflammation, degeneration nor necrosis were detected in tissues adjacent to implants- Changes in nervous fibers, mentioned before, were still retained;
   in 3 months followed by injection, basophilia growth of the CL PAA hydrogel was observed- Its vacuoles were markedly separated from the fascia by thin connective-tissue capsules of collagen and elastic fibers, fibrocytes being in the interspaces- Blood vessels were in the norm- Reaction of penis tissues did not occur (fascia, similar to the control, was presented by the circulatory located marced collagen and elastic fibers, having neither disoders in their structure at the micro- and macrolevels, nervous fibers being in the norm);
   in 6 months the penis forms and sizes in dogs were, by visual evaluation, similar to those observed at the second third days. Histologically, implants were of the whole well-incapsulated dark-blue vacuoles. Capsules consisted of one-two fibrocytes layers and order-lined thin collagen and elastic fibers; calcium salts were neither macroscopically nor microscopically (by von Kos) detected in them. Neither reactive, hemodynamic, degeneratic, necrotic, inflammation nor other changes, including tissue and cell atypism, were found in the implant-adjacent tissues- Nervous tissues, impregnated with silver nitrate, both in the experimental and control animals were virtually identical- In the regional lymphonodes, intra-trabecular and trabecular corpus cavernosum penis, penis veins and lungs, hydrogel particles were not detected;
B. Followed by the CL PAA hydrogel intracavernous injections,
   in 1 day and 7 days, the CL PAA hydrogel, stained with hematoxylin and eosin, was detected as homogenous light-blue vacuoles, which, in seven days, were surrounded by thin connective-tissue capsules; the latters caused a slight shift and compression on corporum cavernosum penis and tunica albuginea. Capsules were constituted of thin, mainly collagen fibers, and one-two layers of fibroblasts-Connective-tissue of corporum cavernosum penis nearby the capsules, were of the usual structure, having markedly distinctive smooth muscles with a minor number of elastic fibers, being of no degeneration and necrosis traits, when studied histochemically or histologically. Minor leukocytes and macrophages accumulation was observed at the surface of the CL PAA hydrogel implants- Intra-trabecular spaces had inconsiderable blood content and slightly swelled endothelium. Lesser arteries and veins were moderately blood-filled, having slightly thickened walls (primarilly, due to endothelium swelling and proliferation of fibroblasts, histocytes and plasmatic cells in the adventitial membranes). Capsules were encircled by the granulation tissue, formed of an insignificant number of the thin-walled newly-formed vessels and different cells, mainly of histogenous origin, i.e.: fibroblasts, histocytes- Giant cells able to resolve foreign elements were not observed. Some nervous fibers, when impregnant and being studied by Spielmeier, showed changes in axis cylinders, myelin membrane and Schwann's cells. Twisting, local swelling and irregular-form thickenings of nervous tissues as well as varicosity and fiber disintegration of axons, spherical and club-shaped enlargements at their ends and definite demielinization region were observed- Reactive proliferation of the Schwann's cells hypertrophied was detected in particular cases;
   in 14 days the leukocytes and macrophages reaction around the CL PAA hydrogel implants became more intensive but the giant cells able to resolve foreign elements were, as mentioned before, not found, however. Connective-tissue capsules around implants were, in some cases, slightly porous and consisted of random-scattered collagen and elastic fibers and young fibroblasts, and in separate cases, were more mature in appearance and consisted mainly of parallel collagen fibers, including elastic fibers and fibroblastic elements. Perifocal granulation tissue was constituted of inconsiderable number of slit-like newly-formed vessels and fibroblasts including a moderate RNA-content and highly-sulfated glycosaminoglycan granules. Nervous tissue changes occured as a local swelling of axial cylinders, their fiber-disintegration, vacuolization, varicosity, hypo- and hyperimpregnation and, occasionally, in local oxoplasm swelling, either along the nervous fibers or at their ends, partial alterations in smoothness of myeline membrane and its disintegration into short and long fragments, which should be considered as a compensatory-adaptive response to compression- In the regional lymphonodes, intertrabecular spaces of corporum cavernosum, penis veins and lungs, CL PAA hydrogel particles were not detected;
   in 1 month around the CL PAA hydrogel implants thin mature connective-tissue capsules were formed; the latter included circle-located collagen and elastic fibers, mature fibroblastic elements being detected among them. Inconsiderable diffusive infiltration, caused by macrophages and plasmatic cells, was detected in the surface-adjacent layers of implants. By their structure, connective-tissue trabeculars of corporum cavernosum did not differ from the control and were covered with the conventional endothelium. Insignificant amount of blood could be found in the inter-trabecular spaces. Walls of the corporum cavernosum veins and arteries were of no marked structural changes. Nervous tissue reaction alterations, as compared with the previous period, were considerably decreased and expressed mainly by the non-uniform regions of thickened and thinned axial cylinders and their hypo- or hyperimpregnation;
   in 3 months the hydrogel was getting a low-dense and basophilic character. Implants were separated from tissues adjacent by the thin-walled capsules with the parallel-located collagen and elastic fibers and an inconsiderable number of fibrocytes between them. Cell elements were not detected at the implants surface. Tissues adjacent and their blood vessels were of the usual structure. Glycosaminoglycans composition of the major connective-tissue substance, fibrous structures and cell elements of connective tissue was virtually identical to the control. Changes in nervous fibers were not observed;
   in 6 months the penis forms and sizes in dogs were visually similar to those observed at the second-seventh days. Histologically, implants had the appearance of solid well-incapsulated dark-blue vacuoles. Capsules consisted of one-two layers of fibrocytes and regular-located thin collagen and elastic fibers; calcium salts, neither macroscopically, nor microscopically by von Kos, were detected in them. No reactive, hemodynamic, degenerative, necrotic, inflammation and other changes, including tissue and cell atypism, were detected in tissues adjacent to the implant. Nervous tissues, impregnanted with silver nitrate, in the experimental and control animals were virtually identical. In the regional lymphonodes, inter-trabecular spaces of the corporum cavernosum and veins of penis as well as in lungs, the CL PAA hydrogel particles were not found.

Similar morphologic data were also obtained in the clinical studies. As a material, bioptat of subcutaneous fat, taken from a healthy volunteer, age of 45, was used; the man, in 6 years before the biopsy, was intradermally injected with 10 ml of a comparative hydrogel, the CL PAA concentration of 8%.

Bioptat was fixed in 10% formaline, dehydrated in alcohols of increasing strength and poored with paraffin. Mounts were stained with hematoxylin and eosin; the collagen fibers were determined by van Gison and the elastic fibers - by Weigert; glycosaminoglycans were determined by toluidine blue under the different pH of solutions, applying the required chemical and enzymatic control; glycoproteide and glycogen concentrations were determined by means of the PAS-reaction by Mac-Manus.

Macroscopically, the bioptat was oval in shape, gentle-elastic by its consistency, light-pink in color without any visual changes, which would be distinctive, if compared with the tissues adjacent.

In microscopic studies all preparations showed presense of the CL PAA hydrogel, stained with hematoxylin and eosine; blue colors obtained were different by their intensity- The implant of the present hydrogel, in bulk, was impregnant by highly vascularized gentle connective tissue, consisted mainly of the regulated collagen and elastic fibers and the ground substance, which included slight admixture of cell elements (as a rule, inactive fibroblasts, since, by the results of staining with toluidine blue at pH=2.8, the traits of neither metachromasy in the form of glycosaminoglycans nor the solitary mononuclear-macrophages were detected in the fibroblasts cytoplasm).

Vessels in the mentioned connective-tissue interlayers were located in groups and their walls were of different thickness having the flat endothelium.

Signs of acute and chronic inflammation, i.e. polymorphonuclear leukocytes, epitheloid cells, giant cells able to resolve foreign bodies and lymphoid-histiocytic infiltrations, were completely absent; traits of allergic reactions in the form of lymphocytes, macrophages and histiocytes as well as of hemodynamic disorders expressed in the vessel plethora, pre-stasis, hemostasis, thrombosis and malignization, e.g., cell- or tissue atypism and cell proliferation, were not observed, too.

Calcium salts were not detected in the mounts - either macro- or microscopically. Alternative, i.e. dystrophic or necrotic changes, were not found.

Fibrous capsule around the implant was absent.

The major method for correcting cosmetic or functional defects of human organism, using the CL PAA biocompatible hydrogel suggested, consists in the following:
based on anamnesis, examination and, as required, laboratory studies, i.e., generally accepted for patients would-be surgically treated (specifically, determination of the individual antibiotic sensitivity), preliminaries, as follows, should be considered, viz.:
   - first, definition of an organ would-be corrected either by its form and size or by the functional efficacy, and
   - second, determination of the volume, tactical measures and regimen (ambulatory or clinical) of the would-be correction;
before the hydrogel suggested is to be injected, local anesthesia, as a rule, should be performed;
the sterile CL PAA hydrogel, additionally impregnated with antibacterial preparations, should be slowly syringed (usually, in two-three stages) into the correction region at a temperature similar to the normal for humans (36-37°C).

The given method is highly effective in mammoplasty (preferably, in aplasia and hyperplasia) and phalloplasty, when the impotency is expressed in poor erection or is a result of agening or injuries happened.

Thus, when applied for mammoplasty, the CL PAA hydrogel, having the preferable concentration of 3.5-6% and the most preferable concentration of 5-6%, depending on the individual anatomic mamma peculiarities, is retromammarily, intracapsulary or/and subfascially injected in the two-three stages; usual dose is 40-160 ml (but not exceeding 200ml) per one mamma for one stage.

Specifically, when applied for phalloplasty,the CL PAA hydrogel, having the preferable concentration of 4.5-6.0% and also the most preferable one of 5%, is, as a rule, intracavernously injected into the three segments along each side of penis in trabecular corpus cevernosum. Total amount of the CL PAA hydrogel as required for one phalloplastic operation, is preferably 40 to 60 ml. The amount for particular case is calculated by the criteria of permissible volume and degree of penis elasticity, excluding possibility of the urethra compression.

The biocompatible hydrogel was clinically tested.

Specifically, it was applied according to the invention for mammoplasty in mammaaplasia and hypoplasia in women.

Individual examples of correcting defects of forms and sizes of mammas are sited below.
(1) Patient L. (Med. history No- 12), yr/birth 1967, being parturiated before.
   Diagnosis: Symmetrical mamma aplasia-
   Correction in three stages (local anesthesia: 0.5% novocain solution, 80 ml at each stage):
   - the first stage (January, 1991) - intramuscular, retromammar and subcapsular injections of 140 ml 6%-CL PAA hydrogel into both mammas;
   - the second stage (March, 1991) - similar injection of the given hydrogel, 40 ml into both mammas
   - the third stage (May, 1991) - similar injection of the given hydrogel, 60 ml into both mammas.

   Result obtained was positive: the patient's mammas, in their shape and size, were in conformity with her physique; their elasticity was similar to the natural soft tissues.
(2) Patient N. (Med- history No. 78), yr/birth 1969, non-parturiated before.
   Diagnosis: Symmetrical mamma hypoplasia.
   Correction: (local anesthesia: 0.5%-novocain solution, 80 ml);
   - the first stage (January, 1994) - intramuscular, retromammar and subcapsular injections of 130 ml 6%-CL PAA hydrogel into both mammas;
   - the second stage (-July, 1994) - the similar injection of the given hydrogel into both mammas.

   Positive result, similar to the above described, was obtained.

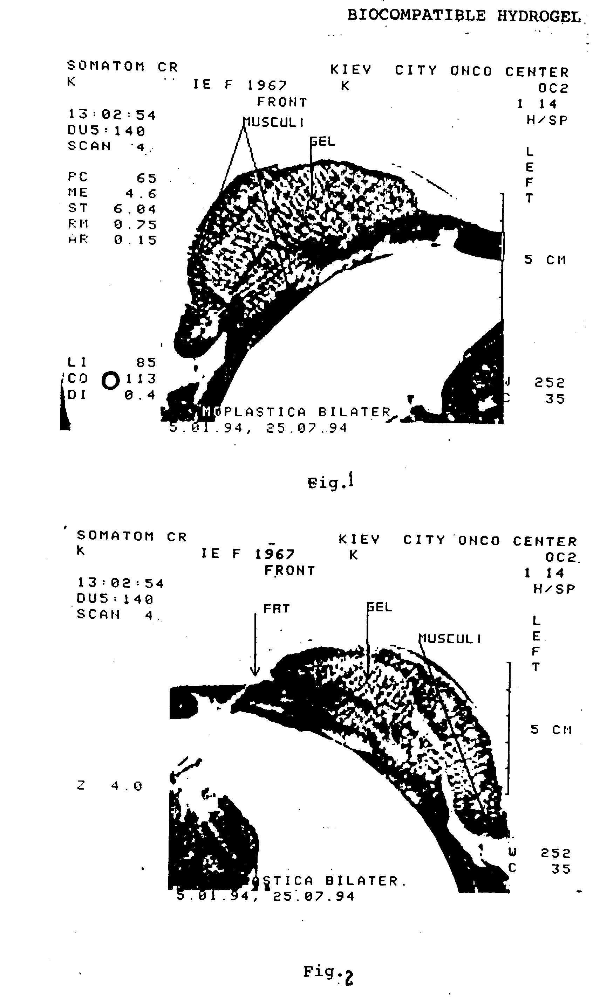

Correction results were additionally evaluated using the chest axial computer tomography; it was carried out at the tomograph "SONATRON CR" of SIEMENS production, FPG; spacing of 8 mm; the mentioned patient was in the "back-lying" position and tomography was performed at the mammas level. Two tomograms of the numerous obtained are shown in the above-mentioned Fig.1 and Fig.2.

As it follows from illustrations, both mammas , as a result of correction, are of regular topography and form. Cutaneous thickness does not exceed 2.0 mm, nipples nad areolas are in the norm (neither deformed nor drawn). Hypoplasized tissue of both mammas is ventrally shifted by the CL PAA hydrogel (different by the density if compared with the former), which is filling the retromammary space (glandula mammaria tissue density is + 3.0 to 4.0, density of the mentioned hydrogel is +4.6 to 7.2 and subcutaneous density is -73 to -96 units Hu).

Glandula mammaria dimensions after correction were as follows:
transversal of 7.4cm dextra and 8.0 sinistra;
longitudal of 5.0cm dextra et sinistra.

Regional lymphonodes were not enlarged, osseous tissues of breast bones and ribs were of normal structure.

Laboratory, experimental and clinical data allow to conclude, that the CL PAA hydrogel suggested is chemically and biologically stable, inert, biocompatible and highly suitable for injectable endoprosthesis.

## Claims

1. Use of a biocompatible hydrogel containing 3.5 to 6.0 % by mass of a polyacrylamide, which is cross-linked with methylene-bis-acrylamide, and pyrogen-free water as dispersion medium for the preparation of an injectable endoprosthesis for mammoplasty or phalloplasty.

## Patentansprüche

1. Verwendung eines biokompatiblen Hydrogels, das 3,5 bis 6,0 Masse-% eines mit Methylen-bis-acrylamid vernetzten Polyacrylamids und pyrogenfreies Wasser als Dispersionsmedium enthält für die Herstellung einer injizierbaren Endoprothese für die Mammo- oder Phalloplastie.

## Revendications

1. Utilisation d'un hydrogel biocompatible contenant 3,5 à 6,0 % en masse d'un polyacrylamide, qui est réticulé par du méthylène-bis-acrylamide, et de l'eau apyrogène comme milieu de dispersion pour la préparation d'une endoprothèse injectable pour une mammoplastie ou une phalloplastie.
